# EUROPEAN PATENT SPECIFICATION

(11) **EP 3 189 867 B1**
(45) Date of publication and mention of the grant of the patent: **16.11.2022**
(21) Application number: 16150280.2
(22) Date of filing: 06.01.2016
(51) Int. Cl.: A61M 5/178, B29C 45/16, B29C 65/48, B29C 65/00, B29C 65/02, F16B 11/00

(54) **METHOD FOR MANUFACTURING PARTS AND ASSEMBLING PARTS TO AN INJECTION DEVICE USING 2-COMPONENT INJECTION MOLDING**
VERFAHREN ZUM HERSTELLEN VON TEILEN UND MONTIEREN VON TEILEN AN EINER EINSPRITZVORRICHTUNG MIT 2-KOMPONENTIGEM SPRITZGIESSEN
PROCÉDÉ DE FABRICATION DE PIÈCES ET ASSEMBLAGE DE PIÈCES POUR UN DISPOSITIF D'INJECTION PAR MOULAGE PAR INJECTION À DEUX COMPOSANTS

(43) Date of publication of application: 12.07.2017
(73) Proprietor: Ypsomed AG, 3401 Burgdorf (CH)
(72) Inventor: Moser, Ulrich, 3412 Heimiswil (CH); Bernhard, Mario, 3400 Burgdorf (CH); Kaufmann, Nadine, 3400 Burgdorf (CH)
(74) Representative: Meier Obertüfer, Jürg

(56) References cited:
- EP-A1- 2 737 889
- EP-A2- 0 718 002
- EP-A2- 1 422 735
- WO-A1-2012/116791
- DE-A1- 10 245 355
- DE-A1-102005 047 511
- DE-U1-202007 014 474

## Description

The present invention relates to a method for manufacturing parts and assemblies of parts for a drug delivery device, particularly for an injection device having a housing that is made of several components that need to be joined together.

Portable drug delivery devices are generally known for the administration of a medicament or medication, for example insulin, growth hormones or drugs used for specific therapeutic applications such as cancer treatment and the like. Such a drug delivery device is typically shaped as a pen which can be held by the user or a health care professional for injecting the drug. The drug delivery devices can be designed as re-usable devices where a cartridge or ampoule is inserted into the device from which the drug is delivered to the patient. After emptying the cartridge, a new and full cartridge is inserted into the device for repeated use. As an alternative a disposable device is used which is discarded once the cartridge is empty.

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

Such delivery devices are made from several parts and comprise a housing that preferably is made of more than one housing part. In WO10115820 a first part of the housing is affixed to a second part of the housing. The first part has selected areas where an adhesive is deposited and outside the selected areas no adhesive is deposited. The first and second part are joined together and the adhesive is activated such that both parts are joined together without the adhesive being displaced or present outside the selected areas. The adhesive is however applied on the first part using printing devices or masking techniques which are cumbersome and expensive. Additionally, it is difficult to produce parts as semi-finished goods that can be stored until used, for example at a different location than the production site of the first part.

In US2005103432, a method is disclosed for producing a plastic part having an adhesive layer that is layered up during an extrusion process. The adhesive layer, preferably a hotmelt adhesive, and the base layer are fabricated using a 2-component extrusion process. The adhesive layer comprises metal particles and is activated by heat using induction. The extruded parts are subsequently used in sheet molding processes in the automotive industry. DE 102 45 355 A1 discloses a method for joining a quick-acting connector to a tube wherein the housing of the connector is fabricated comprising a plastic sheath in a two-component injection molding process. The plastic sheath is then laser-welded to the tube. DE 10 2012 111 743 A1 discloses a container for a medical product wherein the outlet port of the container is equipped with a tamper evident cap. The cap is adhesively attached to the port, wherein the adhesive is applied as a ring to the circumference of the port in a two-component injection molding process. The adhesive ring is then activated by spin welding of the cap. WO 95/21734 discloses a process for fabricating a gluable molded part which comprises a hot-melt type gluing part injection molded to it. The gluing part is then activated by heat before gluing the molded part to the object to which it must be glued.

It is an object of the present invention to provide a method for producing first parts, preferably housing parts, suitable for joining to second parts, preferably second housing parts whereby an adhesive material is applied to the first part or housing part using large scale manufacturing processes such as injection molding. The first and second parts are used for injection devices such as injection pens or infusion devices. The adhesive material is applied to specific regions of the first part only and such that upon joining the first and the second parts, no adhesive material is expelled from the connecting site. It is an additional object of the present invention to provide a reliable and efficient assembly method for the first and the second parts or housing parts. Furthermore, it is an object to produce first parts having the adhesive layer that can be prefabricated using injection molding technology and have a shelf-life such that they can be used for device assembly upon command.

This object is solved by the method according to claim 1. Preferred embodiments are specified in the dependent claims, the description and the figures.

### General description

Delivery devices such as injection pens or infusion pumps are made of several parts that need to be assembled together during manufacturing. Dose delivery devices such as injection pens comprise, for example a dose setting mechanism, a dose delivery mechanism having a piston rod or, in case of a re-usable device a reset mechanism for resetting the piston rod. The mechanisms are normally made of several components that are, for example, made by injection molding of plastics, the assembled components of a subassembly can, for example, be regarded as a part. The mechanisms or parts are typically encompassed by a housing which gives structural support required for operating the injection pen and enables a user to hold and handle the injection device. Such a housing is designed as a single part but often is made of more than one part for ease of assembly, to reduce production costs, or because it is made from different types of materials that cannot be processed in a single step. For example the housing of an injection pen is a tubular shaped main housing part having an aperture for viewing the dosage set by the user and the aperture is closed by a second polymeric material which is transparent. Another example refers to housings that are made of a main housing designed as a tube which holds the delivery mechanism on the inside but the main housing is surrounded by a dose scale drum which is part of the dose setting mechanism. In such case the housing comprises also an outer housing which is also tubular shaped, but having a larger diameter than the inner housing to enclose the dose scale drum. In such case the inner and outer housing are preferably made as two separate parts that are joined or welded together during device assembly. The housing can be also subdivided into two or more parts that need to be joined together along the longitudinal axis of the housing because parts of the drive and/or dose setting mechanisms need to be inserted during assembly in a first housing part and need to be axially and /or rotationally fixed by a second housing part that is welded, joined or adhesively attached to the first part or main housing part. In another example, the delivery device is assembled in sub units, for example a sub-unit A and a sub-unit B that are manufactured at a device company and subsequently delivered to a pharmaceutical company which inserts a carpoule, prefilled syringe or other medicament container in one of the sub-units and performs the final assembly by joining the housings of both sub-units together.

The housing or housing parts can be joined together by mechanical means such as a snap-fit connection, a screw type of connection or a form and/or friction fit connection which implies that both housing parts and elements of the housing parts are in a direct contact to each other. For manufacturing and economic reasons it is preferred to have an adhesive type of connection where the surfaces of both housing parts are not in direct contact with each other but via a third member which acts as an adhesive member which adheres or interacts in a form-fit manner to the surfaces of both housing parts and ensures that mechanically both housing parts behave as a single part. Another reason for selecting adhesive types of connections is because the two housing parts are made from two different materials, the two different materials can be two different types of polymeric materials but one housing part can also be made from metals such as steel, aluminum and the like. One part can, for example also be made from a ceramic material. In case of combining different types of materials, the mechanical attachment of the two parts as described above might be cumbersome, not reliable or expensive and the adhesive type of bonding is a viable alternative.

It is preferred to selectively deposit the adhesive material onto or in the first part, for example a first housing part such that after joining and activation of the adhesive, no material is expelled from the connection site which would require removal of excess of adhesive or deburring of the cured or hardened adhesive requiring additional processing steps. Additionally, there is no risk that the excess of adhesive material enters the mechanics of the pen, thereby potentially blocking dose setting and/or dose delivery. The deposition of the adhesive material on at least one of both parts, for example housing parts is preferably done using injection molding technology since manual application of the adhesive is labor intensive and reduces the reproducibility and/or accuracy.

The adhesive materials used can be selected from, but are not restricted to the group of pressure sensitive adhesives, curable polymeric materials such as polyurethanes, isocyanates, epoxy resins, thermoplastic materials used as hot melt materials, hotmelt materials, acrylic based materials. After application of the adhesive material onto or in the first part, the first housing part can be regarded as a semi-finished good which can be stored and used upon request. The semi-finished first part thus can be joined together with the second housing part in a separate step which can be later in time than the production step of the first part, e.g. the first part has a shelf life. The adhesive material can be activated using heat, ultra-sonic welding techniques, microwaves, inductive heating, infrared heating, visible light, UV light, X-rays or Electron beam irradiation. For example a hot-melt adhesive material is heated, expanded and subsequently joins both parts together after cooling down. The adhesive material can be a single material or blended with an additive. Additives such as metal particles, or organic accelerators facilitate heat generation and/or initiate a chemical curing reaction inside the adhesive layer. Alternatively, the adhesive material is blended with a foaming agent which ensures that the volume of the adhesive layer is expanded upon activation, thereby increasing the efficiency for producing a contact to the surfaces of the first and/or second part that need to be joined together.

The adhesive material can be activated according to one of the methods recited above, the activation time depends on the activation method, the power of the activation source and the geometry of the power source. The activation times are typically in the range between 0.1 and 180 seconds, preferably between 0.2 and 100 seconds, more preferably between 0.3 and 30 seconds for incorporation in mass production processes.

The method for producing first parts that can be joined with second parts can also be applied to different types of injection devices such as motor driven autopens, spring driven auto injectors, patch pumps or patch injectors or infusions pumps.

A method for injection molding at least one first part for an injection device wherein a first molding material is injected into a first injection mold to shape the first part, the first molding material preferably having non adhesive properties. A second molding material is subsequently injected into the first mold containing the first part, whereby the second molding step can be done under different conditions (injection time, temperature or pressure) compared to the first molding step. Alternatively, the first part is transferred from the first mold to a second mold prior to the second molding step and the second material is injected into the second mold. The second molding material is injected around the first molding material of the first part at a location which is intended for adhesive connection between the first part and a second part, whereby the location is in the overlap area between the first and second part once the first and second part have been assembled together either prior to or after the activation of the second molding material. The activation of the second molding material activates the second molding material such that it has adhesive properties that were not present before the activation process. The activation of the second material thus can be used to irreversibly join the first and second part together.

The method for injection molding of a tubular shaped first housing part is disclosed wherein in a first step a first material is injected into a mold and whereby in a subsequent step, the second material or adhesive materials injected into the same mold. Alternatively, the first housing part is injection molded with the first material in a first mold requiring molding conditions (speed, melt-temperature, mold temperature) then the molding of the second adhesive material, which is done in a second mold using different molding conditions. These are so-called two-component injection molding processes. The second material is injected such that it at least partially surrounds the first part and at a specific location which is intended for adhesive connection between the first and the second part such that the location is in the overlap area between the first housing part or first part and the second housing part or second part once the first and second parts or housing parts have been assembled together. Initially the first and second part can be assembled in a reversible manner but which can be transferred into an irreversible bond by activating the second material. After the injection molding process of the second material onto the first part or housing part, the second material is deposited at a specific location and the second material can be shaped in a specific geometry which can be at least one circumferential rim or ring surrounding the first part. Alternatively the second material is deposited as separate dots on the first part. Once the first and second part are joined together, e.g. physically brought together without having a formal or irreversible bond between the two, the second material is activated by heat, light (UV, X-ray), or one of the methods listed above to provide a strong bond between the first and the second parts or housing parts.

The method for injecting a first molding material into a first injection mold whereby the second molding material is injection molded into the first mold. In order to create space or a cavity that can be filled with the second molding material, a part such as an insert a slider, a ring or parts of a ring can be moved axially, radially or rotationally from a first position during the first injection molding of the first material into a second position creating the cavity or space needed for injection molding during the second injection molding step of the second material. The movement of the part is preferably accompanied by the release of the gating or sprue required for injecting the second molding material into the first mold.

The first part and the second part are designed such that they are connectable to each other such that they can be brought together or assembled together to form a reversible connection that also can be disconnected to bring the two parts apart. After the first part and the second part are connected together in a reversible connection, the second material is activated such that an irreversible bond between the two parts exists, e.g. they behave as if they were one part. The irreversible connection is defined here as a connection that can be released or broken only using excessive or brutal forces which are beyond the intended forces to be applied during normal use.

The method for injection molding the first part whereby the first part and the second part are housing parts and the second part has a hollow end with inner dimensions or an inner shape that fits around an end of the first part having outer dimensions or a shape that fits into the hollow end of the second part. After aligning and assembling the first and second part, the outer and inner dimensions of the first and the second part are in a form fit connection prior to the activation of the second material. The first part is, preferably, shaped as a hollow tube but alternatively the first and second parts are box shaped with at least one opening. The tubular shaped second part or housing part can be moved over the first part or housing part thereby creating an overlap between the first and the second part and the second material is preferably injection molded onto the first part in that overlap area which is the location for the intended adhesive connection. Examples of the first parts are the inner housing or inner tube, a dose unit for an injection device, a dose sleeve. Examples of the second housing parts can be an outer tube or a cartridge holder, a scale drum, and insert or a dose window. Alternatively the first and /or second parts can be a piston rod with a flange and the piston present in a cartridge or a prefilled syringe.

The second molding material is preferably injection molded onto or around the first part at the location intended for connection to the second part, e.g. in the overlap area between the first and second part. At the location intended for connection, the second molding material can be present in different geometries or shapes. For example the second molding material is present as at least one circumferential rim or ring surrounding the first part. The circumferential rim or ring can be continuous or discontinuous. Alternatively, the second material is shaped as separate lines or dots on the first part. The second material can be injection molded on top of the surface of the first part or is injected in a recess present at the first part. Alternatively, the second material is injected into holes or pockets present at the surface of the first part.

The method for injection a first housing part, whereby the second molding material is a hot melt, a pressure sensitive adhesive, a plastic material different from the first molding material, a UV curable material, a polyurethane or an epoxy based material. Optionally, the second material is blended with an adhesion promotor which enhances the adhesion between the second molding material and the surface of the first housing part. Examples of adhesion promotors are silanes or epoxy silanes. As an alternative, the second material is blended with e.g. a foaming agent which ensures that the second material expands during activation by one of the means described above. Examples of foaming agents are organic molecules such as n-heptane or pentane. Alternative foaming agents can be, for example, simply water. Alternatively, the second material can be blended with metallic particles such as iron particles to improve the heat conductivity within the second material and/or the inductive heating of the second material by an external source.

The first housing part is preferably made by first injecting the first material followed by injection of the second material into the same mold or in a separate mold. The first molding part having the first and second material is preferably designed or intended to be used as a semi-finished good, e.g. the part is produced first and in a second step that is later in time, the first and second housing parts are joined together.

The location for the intended connecting site between the first housing part and the second housing part is selected such that in the assembled state the location is in the overlap region between the first and the second housing part. The shape of the connection site is, preferably a circumferential rim surrounding the first housing part, the location is preferably filled with the adhesive material such that the outer diameter of the location of the connection site is below the diameter of the second housing part. Preferably, the location of the intended connection site is shaped as at least one circumferential recess that can be filled, for example during the two component injection molding process with the adhesive material. For example, the circumferential recess of the first housing part forms a closed compartment with the walls of the mold and the area surrounding the recess, can for example be elastically deformed by protrusions present in the mold. During the injection molding process, the flow of the second molding material can be restricted to the desired area. At the location of the intended connection site, the connection site can also have different shapes such as a plurality of pockets that can be filled with the adhesive material.

Furthermore the invention defined in claim 1 relates to a method of joining the first and the second housing parts together to form an integrated housing. During joining, also other components of the drive and/or dose setting mechanism can be integrated and/or fixated. The method comprises the steps of providing a first housing part produced after the injection molding process described above and having the intended connection site with the second molding material or adhesive material. Providing a second housing part with an inner diameter or inner dimensions above the outer diameter of the first housing part at the location intended for adhesive connection such that the tubular shaped second housing part fits over the connecting site creating an overlap region required for the adhesive bonding between the two housing parts. The first and second parts or housing parts are subsequently aligned along the longitudinal axis of one or both parts or housing parts and joined together by axially bringing the two parts together. The joining is preferably a reversible joining process, e.g. the two parts can also be disassembled again. The overlap area between the first part and the second part is the area intended for adhesive connection, e.g. the area which is selected for injection molding the second material onto or around the first part. The second molding material is activated to cure, expand, foam, adhesively bond or weld the two housing parts together. The activation of the second molding material comprises a heat treatment. Either before or after the activation step, the first and second housing parts can be rotated with respect to each other to enhance the distribution of the adhesive material

The tubular shaped second housing part is made from a metal such as steel, aluminum, copper or a metal alloy. During or after the joining process activating the second adhesive material, the housing part can be plastically deformed to enhance or fixate the two housing parts with respect to each other until the activation and/or curing process is finished to establish a stable adhesive bond. The plastic deformation can be done by puncturing the outer metal shell of the second housing part with, for example, a pin. The plastic deformation can also be combined with a rotational movement of the second housing part with respect to the first housing part leading to an additional plastic deformation of the outer surface of the first housing part and therewith enhancing the connection between the two housing parts. Alternatively, the second material present on the first part is elastically or plastically deformed during the joining process of the first part and the second part. In that case the first and second part are brought into a press-fit connection prior to the activation process.

The activation means for activating the second material surrounding the first housing part can be applied in several ways. For example, and not according to the invention, heat, UV light, laser light, e-beam, X-rays, infra-red or inductive heating can be applied from the inside of the first housing part. Thus the energy is transferred through the wall of the first housing part to the second material that is activated and initiates the adhesive connection or welding of the first and the second housing part. As an alternative the energy source can be applied from the outside, thus heat, visible light, UV light, e-beam radiation or X-rays are applied from the outside and transmitted through the second housing part before arriving at the second material. Alternatively the first and/or second housing parts itself can be used as a means to generate the energy required for activation or initiation of the second material. For example the second housing part can be brought in a high frequency vibrational mode to generate friction between the inner surface of the second housing part and the adhesive material thereby generating heat to activate and/or melt the adhesive layer. Alternatively, in case the second housing part is an electrical conductor, a voltage can be applied on the second housing part and due to the internal resistance, heat is generated. In another example inductive currents, namely eddy currents, are used to heat the second housing part. Thus, according to the invention as defined in claim 1, the heat is transferred from the outside of the second housing part through the metal to the location intended for adhesive connection or the second part is heated by inductive heating.

The assembling of the first part and the second part prior to or during activation can be used to reduce the play, for example axial play, between the two parts, The second part is moved over an end of the first part to create the overlap area but the axial movement of the second part can be halted in several ways. For example an axial and/or radial stop is present at the first part which stops the axial movement of the second part. In a subsequent step, the second material is activated to form an irreversible bonding between the first and second part. Alternatively, the second part is moved along a longitudinal axis towards the first part to create an overlap area between the first part and the second part. However the first and second parts as such can be assemblies of more than one component. In such a case components of the assemblies of the first and second part can abut each other whereby an axial force is transmitted from the second part to the first part assemblies. The axial movement can be stopped at a certain force level and the first and second part are joined together by activating the second material. The first part and second part assemblies have thus been brought together in a force or position controlled manner prior to irreversible fixation.

The first housing part is preferably made from a polymeric material for example and not limited to polyethylene (LDPE, HDPE, LLDPE, UHMWPE) , polyoxymethylene ( POM), cycloolefinic (co)polymer (COC, COP), polysulfones (PPSU, PSU), ABS, polyamides ( PA 4-6, PA 6, PA 6-6, PA-12 or amorphous polyamides), polycarbonate (PC), isotactic polypropylene (i-PP), polystyrene, liquid-crystalline polymers (LCP), PBT, ABS or PC/ABS blends. The polymeric materials can be compounded with short or long fibres to increase the mechanical properties. Glasfibres or carbon fibers are preferred for the reinforcement. As an alternative, the first housing part is made from a metal or ceramic material that also can be injection molded using metal injection molding (MIM).

The second material or adhesive material used as a hot melt can be selected from, but is not restricted to ethylenevinylacetate (EVA), polyolefins (LDPE), atactic polypropylene (a-PP) Polybutadiene, Thermoplastic elastomers like TPU, amorphous polyolefins or oxidized polyolefins.

The second molding material is applied onto the first housing part using injection molding.

Alternatively, but not according to the invention, the second molding material is applied manually using a hot-melt glue gun, or the second molding material is applied using a semi-automated process whereby the first housing part is rotated in front of the nozzle of a hot-melt glue gun.

Alternatively, but not according to the invention, the second molding material is applied on the second part or housing part instead of on the first part or first housing, or the second molding material is applied on both parts. Several preferred embodiments of the invention are described above. Specific embodiments of the invention are described below and visualized in the Figures:

### Legends to the Figures

Figure 1: First housing part in an injection mold supported by a core, the second material can be molded through the vertical injection channel,
Figure 2: First housing part after the second material (adhesive) has been molded,
Figure 3: Alignment of the second housing part and the first housing part before assembly,

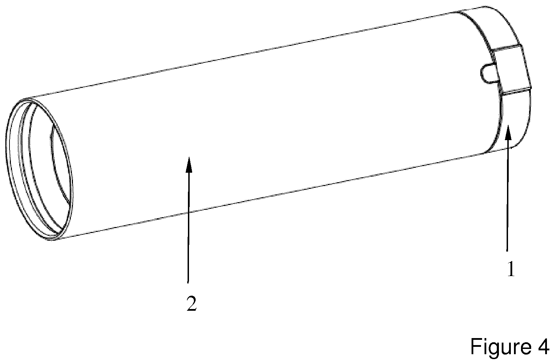

Figure 4: First and second housing part after the joining/welding process,
Figure 5: Force displacement curves,
Figure 6: Break loose forces for the first and second housing part assembly. The adhesive material on the first housing part was manually injected into a mold and subsequently the two parts were joined together using inductive heating,
Figure 7: Break loose forces for the first and second housing part assembly. The adhesive material on the first housing part was injection molded and subsequently the two parts were joined together using conductive heating. Prior to testing, the parts were subjected to accelerated ageing in humid, heat or cold conditions.

### Detailed description of the invention

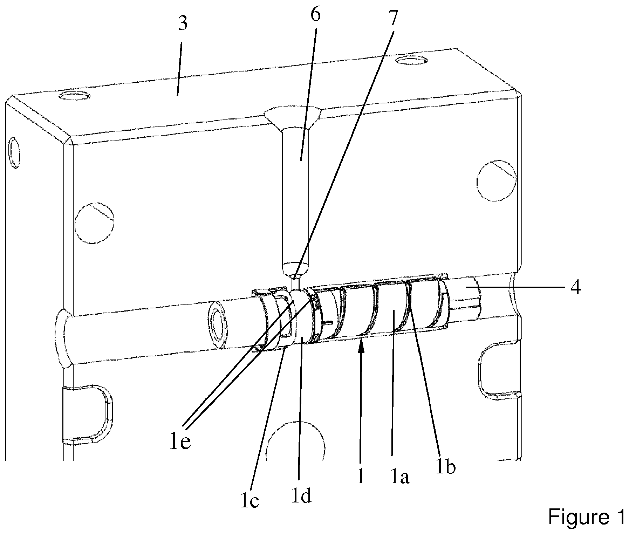

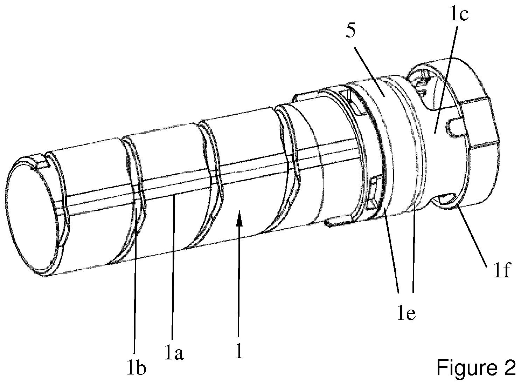

In Figure 1, a tubular shaped first housing part (1) is shown inside a mold (3). The first housing part (1) has a cylindrical section (1a) with an outer groove (1b) that can engage a threading of, for example, a scale drum of an injection pen (Figure 2). The threaded section (1a) is adjoined by a tubular section (1c) having a larger diameter then the cylindrical section with the groove (1b). The first housing part shown in the injection mold (3) is supported by a core (4) which is made from a metal to support the first housing part (1) during the injection molding process and to facilitate the handling. In this example, the first housing part has been injection molded in a first molding process having a first set of processing parameters (melt-temperature, mold temperature, cycle times) adjusted to the needs of the first material used, in the example shown a glass fiber reinforced polyamide (Grivory, EMS Chemie). After the first molding process, the first housing part (1) with core (4) is inserted in the second mold (3) for applying the second material or adhesive material (5). The mold (3) has a vertical channel (6) for injecting the second material (5) onto or surrounding the first part (1). Tubular section (1c) comprises a circumferential recess (1d) with two side walls (1e) having a larger diameter compared to the recessed section (1d). The first housing part (1) is axially positioned, for example using the core (4), such that the recessed section is in front of the nozzle (7) at the end of the injection channel (6). After closing the mold (3) with a second mold piece (not shown), the second material (5) is injected to fill the recessed section (1d) of the first housing part (1). After ejection from the mold (3) and cooling, the first housing part is ready to be used as a semi-finished good (Figure 2), which can be stored and/or transported prior to assembly with the second housing part (2).

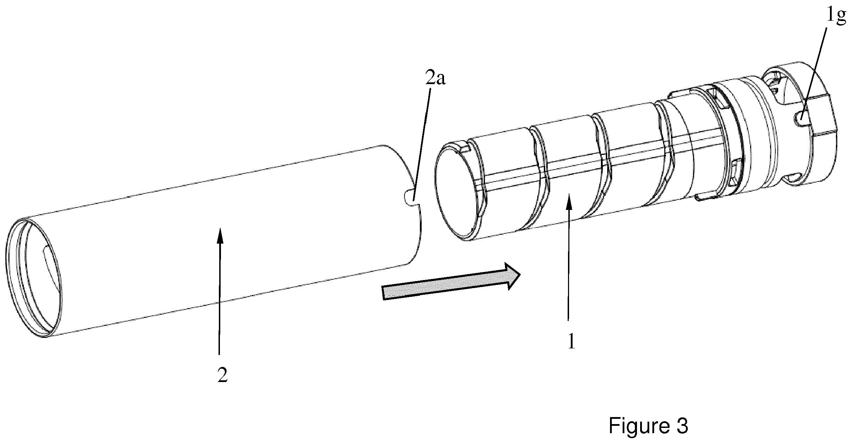

The method for joining the first housing part (1) and the second housing part (2) is shown in Figure 3. The second housing part (2) in this example is made from aluminum. The inner diameter of the second housing part (2) is larger than the outer diameter of the circumferential side walls (1e) that border the recessed section. The central axis of the first and second housing parts are aligned and the second housing (2) part is moved over the cylindrical section (1a) of the first housing part and covers the circumferential recess (1d) containing the second material (5). The axial movement of the second housing part (2) is halted at a rim (1f) at the end of, and attached to the first housing part (1) having an outer diameter at least equal to the inner diameter of the second housing part (2). Optionally, the second housing part (2) has an indention (2a) which fits into a protrusion (1g) of the circumferential rim (1f) to rotationally secure the first housing part (1) with the second housing part (2). This procedure can also be reversed, e.g. the first housing part (1) moves towards the second housing part (2) or both part move towards each other until both parts are in a rotationally and axially secured position with respect to each other. Once the first and the second housing parts are assembled, the recessed section (1d) containing the second material (5) is completely covered by the second housing part (2).

In the next step, the first and the second housing parts are joined together. In the example shown, a hot melt material is used as the second material (5) which, for adhering to the inner wall of the second housing part (2) needs to be heated. Preferably, the heat is applied locally to prevent any deformation or warpage of the first housing part (1) due to internal stresses present from the first injection molding process. Preferably, the metal sheet of the second housing part (2) is locally heated using inductive heating or directly contacted to a hot ring surrounding the second housing part at the location of the recessed section (1d). Alternatively, a hot air gun can be used for heating. For inductive heating, a separate coil surrounds the second housing part (2) and the oscillating electromagnetic field in the coil inductively and locally heats the metal of the second housing part (2). The heat is transferred to the second material 5 which melts /and or expands to create an adhesive joint between the two housing parts after cooling down. Alternatively, the second housing part (2) is locally heated using laser light and/or infrared light and/or by irradiation with an electronic beam. In yet another alternative, the second and/or first housing part is ultrasonically vibrated and the friction between the contact areas of the second material (5) in the recessed area with the inner wall of the second housing part generates the heat required for melting the hot melt material. The ultrasonic vibration can be along the longitudinal axis of the housings or along the circumference.

In an alternative embodiment, the circumferential recess (1f) is filled with a tape of polymeric material. For example a HDPE tape, which has been stretched at an elevated temperature but below the melting temperature of the polymer, for example at 130°C, preferably at 120°C or more preferably at 110°C. During stretching, the tape elongates and the polymer molecules are oriented towards the orientation direction. After stretching the tape is rapidly cooled down to freeze in the orientation. Such a tape material is applied in the recessed section (1f) of the first housing part (1). After assembly, the tape is sandwiched between the two housing parts and local heating initiates the shape memory effect of the polymer, e.g., the tape shrinks and the thickness increases thereby joining the two housing parts. For this process, the second injection molding step is not required.

To increase the efficiency of the welding or joining of the first and the second housing part in above described embodiments, the inner surface of the second housing part and/or the outer surface of the first part can be roughened and/or structured (for example by sandblasting) to increase the bonding between the second material (5) and the second housing part (5).

The methods presented here ensure that no adhesive material is distributed outside the desired areas due to specific and local application of the adhesive material (5) using injection molding techniques in combination with the local heating for activating the adhesive material. Due to the absence of macro motions between the first and the second housing parts during welding, no adhesive material is distributed outside the desired area or into the mechanics of the injection pen.

An example of the force controlled approach between the first and second part before irreversible joining is described below. As an example, for illustrative purposes only, the joining of a cartridge holder (second part) containing a cartridge to a dose setting and drive mechanism enclosed in a housing (first part) is discussed. The cartridge holder holds a cartridge with a medication. The cartridge has a tubular shape closed with a septum for connecting to a needle on one side and a rubber stopper on the opposite or open side of the tube. Between the septum and the stopper the medication is present in the cartridge. The dose setting and drive mechanism has a piston rod with a flange at the end and the drive mechanism advances the piston rod such that in the assembled device the flange of the piston rod abuts the stopper of the cartridge and advancement of the piston rod expels medication from the cartridge through the needle. During assembly of the device it can be essential that the flange of the piston rod already abuts the stopper of the cartridge, e.g. without play between the flange and the stopper. As a result, the user does not have to prime the device in an initial step to establish the contact between the cartridge and the flange of the piston rod. One way of assembling the device is to approach the assembly of the cartridge holder (second part) containing the cartridge towards the housing (first part) holding the drive mechanism including the piston rod. Once the stopper in the cartridge abuts the flange at the end of the piston rod, an axial force is exerted onto the second part (housing) that contains the drive mechanism. Thus in a force controlled manner the two parts can be brought together prior to fixation by activating the second material present on the first part. The force can be selected as low as to prevent the stopper from moving in the cartridge, e.g. the contact force must be below the break loose force of the stopper in the cartridge and thus typically below 8 N. Thus a contact force is preferably selected below 4N, more preferably below 2N.

### Further embodiments are exemplified below:

### Materials used:

The materials used as second molding material or adhesive material are listed in Table 1:

**Table 1: Materials used as adhesive material:**

| | **LT 110** | **SikaMelt 9171** | **Technomelt PA653** |
|---|---|---|---|
| Manufacturer | UHU | Sika | Henkel |
| Material | EVA-Copol | Polyolefin | Polyamid |
| Softening Temp [°C] | 100160160 | | |
| Processing Temp [°C] | 110 | 170-190 | 180-230 |

The first housing part is made from a glass fiber reinforced polyamide (Grilamid, EMS Chemie) and the second housing part is made from aluminum. In the examples shown below, the first housing part is first injected in a separate molding.

### Techniques used to apply the adhesive materials onto the first housing part:

A simple hot melt gun (UHU) was used for the test were the second material was manually applied onto the selected region of the first housing part. In a second step, the hot melt guns were assisted with pressurized air (6 Bar, Sika) to assist the flow of material. The assisted hot gun was also used in combination with a mold to perform a manually operated injection molding process. Finally, injection molding was done using an Arburg machine equipped with a 15mm diameter feeding screw

### Heating equipment used to join the first and second housing parts

After joining the first and second housing parts, the assembly was inserted in the central ring of an ISG 2202 DIEBOLD inductive heater (7.5 kW) and heated for 2 to 5 seconds. The temperature of the aluminum of the second housing was 70°C after 2second, 105°C after 3 seconds, 140°C after 4 seconds and 170°C after 5 seconds. The inductive heating times were between 0.1 and 10 seconds, preferably between 0.5 and 8 seconds, more preferably between 1 and 5 seconds.

In another approach, heat conduction was used. The assembly of the first and second housing parts were inserted in a cylindrical metal block with an aperture fitting the outer diameter of the second housing part. The metal block is heating using heating wires to a constant temperature ranging between 100°C and 1000°C, preferably between 150°C and 750°C, more preferably between 200°C and 500°C. The assembly was inserted into the heating block for times ranging between 0.1 and 300 seconds, preferably between 1 second and 200 seconds, more preferably between 5 seconds and 60 seconds. The specific combination between the temperature of the metal block contacting the second housing parts and contact time ensures processing times that are suitable for large scale manufacturing processes.

### Testing of the joined first and second housing parts:

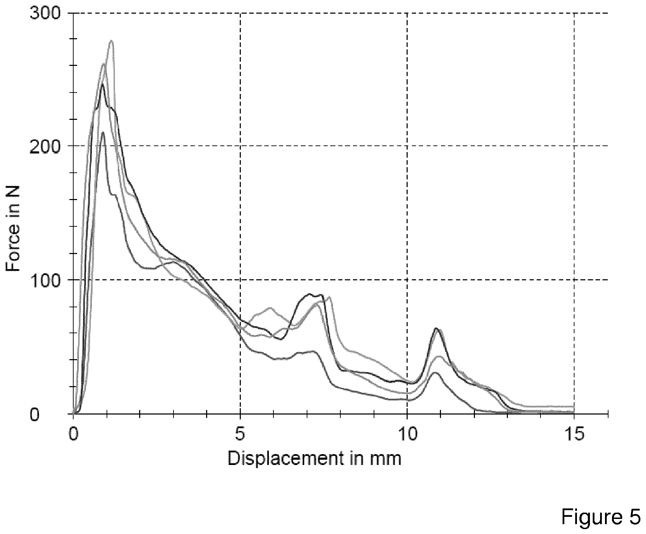

A hole was drilled in the aluminum second housing part and connected to a fixture of the upper traverse of a Zwick Roll Zwicky 2.5 TN tensile tester. The upper traverse was lowered at a test speed of 20 mm/min and force displacement curves were recorded. The peak of the force displacement curves was used a break loose force between the first and second housing part. The values were averaged over n=4 test samples. A typical example of the force displacement curves is shown in Figure 5, first the force increases to a peak value were the adhesive bonding between the first and second housing part fails and subsequently the force decreases. A minimum requirement for the axial break loose force of the second housing part and the second housing part of 90 N was defined based on trials whereby test person tried to bring a maximum axial compressive or tensile force onto an injection pen.

### Preconditioning in a climate chamber:

Test sample were preconditioned for 96 hours at 3 different preconditions: humidity, heat and cold. Details are listed in Table 2:

**Table 2: Preconditioning of test samples**

| Condition | Details |
|---|---|
| Humidity | 40 +/- 2°C, 93+/- 5% rel. humidity |
| Heat | 70 +/- 2°C, 50 +/-10% rel. humidity |
| Cold | -40 +/- 3°C |

### Example 1: Manual application of the adhesive material using a hot gun

The LT110 melt gun from UHU was used to manually apply second material (5) onto the circumferential recess (1d) of the first housing part (1). After cooling, the first housing part (1) with the adhesive layer and the second housing part (2) were assembled and heated for 2 seconds using the inductive heating coil. The axial break loose forces under compression were tested (n=4) with an average of 152 N and a standard deviation of 81N. The standard deviation is relatively large and break loose forces as low as 40N were measured which is below the requirement of 90 N, thus showing that the manual application with the hot gun but without a mold results in a relatively large variation of the test results.

### Example 2: Manual application using a mold

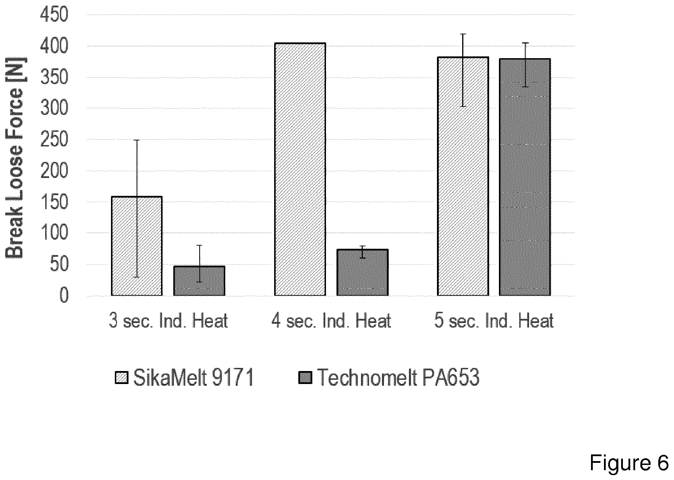

The Sikamelt 9171 and Technomelt 653 hot melt materials were applied using the pressurized air-assisted hot melt gun. The mold is comparable to the configuration shown in Figure 1. The circumferential recess (1) of the first housing part (1) could not be completely filled at the maximum pressure of 6 bar and therefore the adhesive material was applied in two steps. After a first injection step, the first housing part was removed from the mold, rotated such that the non-injected part of the circumferential recess (1d) was facing the injection nozzle and a second injection step using the pressurized gun was used. After the two manual injection processes, the first housing part was removed from the mold and assembled with the aluminum second housing part (2). The second housing part was inductively heated for 2s, 3s and 4 s respectively. After cooling down, the break loose forces were measured using the Zwick tensile tester and the results are presented in Figure 6.

For the Sikamelt9171 hot melt adhesive, after 3 seconds of adhesive heating the standard deviation of the force values is relatively large showing a non-homogeneous adhesive bond between the first(1) and second(2) housing parts. After 4 seconds inductive heating, the break loose forces are at least 400N for all test samples, the test was stopped at this force value. After 5 second heating the average break loose force is still close to 400 N with a low standard deviation. For Sikamelt, a reliable bond with a break loose force far above the requirement of 90 N was achieved at 4 or 5 seconds inductive heating. For the Technomelt PA653, 3 or 4 seconds heating does not result in a good bonding between the two housing parts. After 5 seconds inductive heating of the aluminium, a good bond strength of almost 400 N was achieved for Technomelt. These experiments show that the controlled, but manual injection of the adhesive material results in a reliable bond strength between the first and second housing parts once the correct timing for the subsequent inductive heating is chosen. The heating times are in the range of a few seconds, preferably between 1 and 5 seconds and more preferably 4 to 5 seconds. These heating times for the inductive heating allow for cycle times that can be integrated in large volume manufacturing processes.

### Example 3: Injection molding of the adhesive material:

The first housing part (1) was injection molded first and then inserted into a second mold, comparable to the mold shown in Figure 1. The Sikamelt 9171 was extruded at a barrel temperature ranging between 140° and 200°C, preferably between 150°C and 180°C and more preferably at 160°C. Sikamelt was injected with an injection pressure ranging between 300 and 400 Bar, more preferable between 350 and 390 bar and most preferable at 380 bar. The mold was at a Temperature of 30°C.

The Technomelt PA653 was extruded at a temperature between 150 and 160° with a pressure ranging between 300 and 500 bar, more preferable at a pressure between 320 and 450 bar. The mold temperature was in the range between 25 and 30°C.

After the injection molding process, the first housing parts (1) were removed and assembled with the second housing parts (2). The assembly was heated for 3 minutes in a metal block that was heated at a constant temperature of 240°C, the heat was transferred due to conduction from the metal block to the aluminums housing and finally to the hot melt material present in the recessed area (1d).

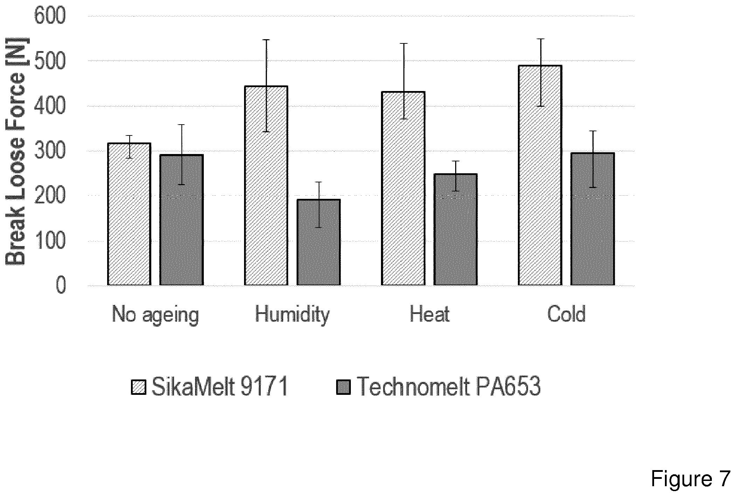

After the welding process, the parts were subjected to a climate chamber to accelerate the ageing of the bonded parts and the results are presented in Figure 7. All samples, whether aged or non-aged prior to the testing fulfilled the requirement of a minimum break loose force of 90 N. The variance of the test data was low enough that none of the test samples had a break loose force below the minimum requirement of 90 N. For the Sikamelt 9171, the ageing under humid, heat or cold conditions all improved the break loose values from 300N to above 400 N. The polyolefin based adhesive improves the bonding strength, independent whether aged in heat, cold or humidity. The polyamide based Technomelt has a lower average break loose force when pretreated in humid or heat and is at the same break loose level as the non-treated parts. Although all parts fulfill the minimum requirement of 90N break loose force, it appears that the polyamide based bonding material is more affected by the accelerated ageing albeit none of the test values is below the minimum requirement. This experiment shows that a variety of hot melt materials can be processed using injection molding techniques and that in a subsequent step two parts may be joined together adequately.

**Reference signs**

| | | | |
|---|---|---|---|
| 1 | First housing part | 2 | Second housing part |
| 1a | Cylindrical section | 2a | Indention |
| 1b | Groove | 3 | Mold |
| 1c | Tubular section | 4 | Core, support |
| 1d | Circumferential recess | 5 | Second material, adhesive |
| 1e | Side walls | 6 | Injection channel |
| 1f | Rim | 7 | Nozzle |
| 1g | Protrusion | | |

## Claims

1. A method for injection molding at least one first housing part (1) and for joining at least two housing parts of a drug injection device comprising the steps of:
providing a first housing part (1) by
injecting a first molding material into a first injection mold (3) to shape the first housing part (1) whereby the first molding material is a plastic material having non-adhesive properties,
providing a second molding material (5) having adhesive properties upon activation,
injecting the second molding material (5) into the first injection mold (3) containing the first housing part (1) or into a second injection mold after the first housing part (1) has been transferred from the first mold to the second mold, wherein the second molding material (5) is injected onto or around the first housing part (1) at a location which is intended for adhesive connection between the first housing part (1) and a second housing part (2), whereby the location is in the overlap area between the first part housing (1) and the second housing part (2) once the first housing part (1) and second housing part (2) have been joined together,
providing the second housing part (2), wherein the inner dimensions of the second housing part (2) are larger than the outer dimensions of the first housing part (1),
aligning the first and second housing parts (1,2) along a common central axis,
assembling the first housing part (1) and the second housing part (2) by axially moving the second housing part (2) over the housing first part (1) thereby covering the location intended for adhesive connection,
activation of the second molding (5) material having the adhesive properties to join the first housing part (1) and the second housing part (2), whereby the activation of the second molding material comprises heating, and whereby the second housing part (2) is made from a metal, preferably
aluminum and whereby the heat is conductively transferred from the outside of the second housing part through the metal to the location intended for adhesive connection or whereby the second housing part (2) is heated by inductive heating.

2. The method according to claim 1, whereby during assembling the first housing part (1) and the second housing part (2), the second housing part (2) part is moved over the first housing part against a stop present on the first housing part (1) to eliminate play between the first and second housing parts (1, 2) or until a certain axial force is applied onto the first housing part (1), preferably to eliminate play between the first and second housing part, prior to the activation of the second molding material (5).

3. The method according to claim 1 whereby during assembling the first part(1) and the second part(2) the second molding material (5) is elastically and/or plastically deformed before the activation of the second molding material (5).

4. An injection device for injecting or infusing a dose of medication which has been produced according to one of the previous claims

## Patentansprüche

1. Verfahren zum Spritzgießen mindestens eines ersten Gehäuseteils (1) und zum Aneinanderfügen von mindestens zwei Gehäuseteilen einer Medikamenteninjektionsvorrichtung, das die folgenden Schritte umfasst:
Bereitstellen eines ersten Gehäuseteils (1) durch Spritzen eines ersten Formmaterials in eine erste Spritzgussform (3), um das erste Gehäuseteil (1) auszuformen, wodurch das erste Formmaterial ein Kunststoffmaterial mit nicht klebenden Eigenschaften ist,
Bereitstellen eines zweiten Formmaterials (5), das nach Aktivierung klebende Eigenschaften aufweist,
Spritzen des zweiten Formmaterials (5) in die erste Spritzgussform (3), die das erste Gehäuseteil (1) enthält, oder in eine zweite Spritzgussform, nachdem das erste Gehäuseteil (1) aus der ersten Form zur zweiten Form übertragen wurde, wobei das zweite Formmaterial (5) an einer Stelle, die als eine klebende Verbindung zwischen dem ersten Gehäuseteil (1) und einem zweiten Gehäuseteil (2) vorgesehen ist, auf oder um das erste Gehäuseteil (1) gespritzt wird, wodurch die Stelle im Überlappungsbereich zwischen dem ersten Gehäuseteil (1) und dem zweiten Gehäuseteil (2) liegt, nachdem das erste Gehäuseteil (1) und das zweite Gehäuseteil (2) aneinandergefügt wurden,
Bereitstellen des zweiten Gehäuseteils (2), wobei die Innenabmessungen des zweiten Gehäuseteils (2) größer sind als die Außenabmessungen des ersten Gehäuseteils (1),
Ausrichten des ersten und des zweiten Gehäuseteils (1, 2) entlang einer gemeinsamen Mittelachse,
Zusammensetzen des ersten Gehäuseteils (1) und des zweiten Gehäuseteils (2) durch axiales Bewegen des zweiten Gehäuseteils (2) über das erste Gehäuseteil (1), wodurch die Stelle, die für die klebende Verbindung vorgesehen ist, abgedeckt wird,
Aktivierung des zweiten Formmaterials (5), das die klebenden Eigenschaften aufweist, um das erste Gehäuseteil (1) und das zweite Gehäuseteil (2) aneinanderzufügen, wodurch die Aktivierung des zweiten Formmaterials ein Erwärmen umfasst und wodurch das zweite Gehäuseteil (2) aus einem Metall, vorzugsweise Aluminium, besteht und wodurch die Wärme von der Außenseite des zweiten Gehäuseteils durch das Metall zu der Stelle, die für die klebende Verbindung vorgesehen ist, leitend übertragen wird oder wodurch das zweite Gehäuseteil (2) durch induktives Erwärmen erwärmt wird.

2. Verfahren nach Anspruch 1, wodurch während des Zusammensetzens des ersten Gehäuseteils (1) und des zweiten Gehäuseteils (2) das zweite Gehäuseteil (2) über das erste Gehäuseteil gegen einen Anschlag, der sich am ersten Gehäuseteil (1) befindet, bewegt wird, um ein Spiel zwischen dem ersten und dem zweiten Gehäuseteil (1, 2) zu beseitigen oder bis eine gewisse Axialkraft an das erste Gehäuseteil (1) angelegt ist, vorzugsweise um ein Spiel zwischen dem ersten und dem zweiten Gehäuseteil vor der Aktivierung des zweiten Formmaterials (5) zu beseitigen.

3. Verfahren nach Anspruch 1, wodurch während des Zusammensetzens des ersten Teils (1) und des zweiten Teils (2) das zweite Formmaterial (5) vor der Aktivierung des zweiten Formmaterials (5) elastisch und/oder plastisch verformt wird.

4. Injektionsvorrichtung zum Injizieren oder Infundieren einer Medikamentendosis, die nach einem der vorhergehenden Ansprüche hergestellt wurde.

## Revendications

1. Procédé pour mouler par injection au moins une première partie de boîtier (1) et pour assembler au moins deux parties de boîtier d'un dispositif d'injection de médicament comprenant les étapes consistant à :
fournir une première partie de boîtier (1) en injectant un premier matériau de moulage dans un premier moule à injection (3) pour former la première partie de boîtier (1), moyennant quoi le premier matériau de moulage est une matière plastique présentant des propriétés non-adhésives,
fournir un second matériau de moulage (5) présentant des propriétés adhésives suite à l'activation,
injecter un second matériau de moulage (5) dans le premier moule à injection (3) contenant la première partie de boîtier (1) ou dans un second moule à injection après que la première partie de boîtier (1) a été transférée du premier moule au second moule, dans lequel le second matériau de moulage (5) est injecté sur ou autour de la première partie de boîtier (1) à un emplacement qui est prévu pour le raccordement adhésif entre la première partie de boîtier (1) et une seconde partie de boîtier (2), moyennant quoi l'emplacement est dans la zone de chevauchement entre la première partie de boîtier (1) et la seconde partie de boîtier (2) une fois que la première partie de boîtier (1) et la seconde partie de boîtier (2) ont été assemblées ensemble,
fournir la seconde partie de boîtier (2), dans lequel les dimensions internes de la seconde partie de boîtier (2) sont supérieures aux dimensions externes de la première partie de boîtier (1),
aligner les première et seconde parties de boîtier (1, 2) le long d'un axe central commun,
assembler la première partie de boîtier (1) et la seconde partie de boîtier (2) en déplaçant de manière axiale la seconde partie de boîtier (2) sur la première partie de boîtier (1) recouvrant ainsi l'emplacement prévu pour le raccordement adhésif,
activer le second matériau de moulage (5) ayant les propriétés adhésives pour assembler la première partie de boîtier (1) et la seconde partie de boîtier (2), moyennant quoi l'activation du second matériau de moulage comprend le chauffage, et moyennant quoi la seconde partie de boîtier (2) est réalisée à partir d'un métal, de préférence de l'aluminium et moyennant quoi la chaleur est transférée, par conduction, de l'extérieur de la seconde partie de boîtier par le biais du métal à l'emplacement prévu pour le raccordement adhésif ou moyennant quoi la seconde partie de boîtier (2) est chauffée par chauffage par induction.

2. Procédé selon la revendication 1, moyennant quoi pendant l'assemblage de la première partie de boîtier (1) et de la seconde partie de boîtier (2), la seconde partie de boîtier (2) est déplacée sur la première partie de boîtier contre une butée présente sur la première partie de boîtier (1) pour supprimer le jeu entre les première et seconde parties de boîtier (1, 2) ou jusqu'à ce qu'une certaine force axiale soit appliquée sur la première partie de boîtier (1), de préférence pour supprimer le jeu entre les première et seconde parties de boîtier, avant l'activation du second matériau de moulage (5).

3. Système (10) selon la revendication 1, moyennant quoi pendant l'assemblage de la première partie (1) et de la seconde partie (2), le second matériau de moulage (5) est élastiquement et/ou plastiquement déformé avant l'activation du second matériau de moulage (5).

4. Dispositif d'injection pour injecter ou perfuser une dose de médicament qui a été produit selon l'une des revendications précédentes.
